# EUROPÄISCHE PATENTSCHRIFT

(11) **EP 2 647 330 B1**
(45) Veröffentlichungstag und Bekanntmachung des Hinweises auf die Patenterteilung: **12.09.2018**
(21) Anmeldenummer: 13160604.8
(22) Anmeldetag: 22.03.2013
(51) Int. Cl.: A61B 1/00

(54) **Schutzhülse für ein ein Endoskoprohr aufweisendes Endoskop**
Protective shell for an endoscope with an endoscope tube
Douille de protection pour un endoscope présentant un tube d'endoscope

(30) Priorität: 04.04.2012 DE 102012205598
(43) Veröffentlichungstag der Anmeldung: 09.10.2013
(73) Patentinhaber: Henke-Sass, Wolf GmbH, 78532 Tuttlingen (DE)
(72) Erfinder: Rehe, Oliver, 78532 Tuttlingen (DE)
(74) Vertreter: Patentanwälte Geyer, Fehners & Partner mbB

(56) Entgegenhaltungen:
- WO-A2-00/57770
- DE-B3-102009 049 143
- US-A- 3 809 072
- US-A- 5 693 043

## Beschreibung

Die vorliegende Erfindung betrifft eine Schutzhülse für ein ein Endoskoprohr aufweisendes Endoskop mit den Merkmalen des Oberbegriffs des Anspruchs 1.

Eine solche Schutzhülle ist z. B. aus der WO 00/57770 A2 und der US 5,693,045 bekannt.

Endoskope werden häufig zur Visualisierung eines Operationsbereiches eingesetzt. Dabei kann es leicht zu mechanischen Beschädigungen des Endoskopes kommen, wenn ein unerwünschter Kontakt mit einem Operationsinstrument auftritt. Um dies zu verhindern, kann eine Schutzhülse mit einem Deckglas vorgesehen werden, so daß lediglich die Schutzhülse und nicht das Endoskop beschädigt wird.

Da der Operationsbereich häufig mit einer Kochsalzlösung gefüllt wird, tritt an dem Übergang zwischen der Kochsalzlösung und dem Deckglas der Schutzhülse Brechung auf, die zu einer Verschlechterung der Abbildungseigenschaften des Endoskopes führen. Insbesondere kann z.B. der maximale Bildwinkel der Abbildungsoptik verringert werden.

Ausgehend hiervon ist es daher Aufgabe der Erfindung, eine verbesserte Schutzhülse für ein ein Endoskoprohr aufweisendes Endoskop zur Verfügung zu stellen.

Erfindungsgemäß wird die Aufgabe durch eine Schutzhülse für ein Endoskoprohr gemäss Anspruch 1 gelöst. Dadurch treten in vorteilhafter Weise die von jedem Objektpunkt ausgehenden und für die Bilderzeugung benutzten Lichtstrahlen senkrecht bzw. im wesentlichen senkrecht durch das Deckglas hindurch, so daß das Deckglas keine oder nur eine sehr geringe Brechung bewirkt. Als Folge davon ist der Einfluß des Mediums an der Außenseite des Deckglases praktisch vernachlässigbar, so daß z.B. der Bildwinkel eines Endoskopes mit aufgesetzter Schutzhülse in Luft und in einer Kochsalzlösung praktisch gleich bleibt. Somit schützt die erfindungsgemäße Schutzhülse einerseits das Endoskop und führt andererseits dazu, daß die optischen Abbildungseigenschaften der Abbildungsoptik des Endoskopes nicht verschlechtert werden, wenn das Endoskop mit aufgesetzter Schutzhülse in einem flüssigen Medium eingesetzt wird.

Aufgrund des transparenten Bereiches tritt, wenn Beleuchtungslicht senkrecht oder im wesentlichen senkrecht durch den transparenten Bereich hindurchtritt, in vorteilhafter Weise keine oder eine im wesentlichen vernachlässigbare Brechung unabhängig vom an der Außenseite des transparenten Bereiches vorhandenen Medium auf. Damit kann z. B. sichergestellt werden, daß selbst bei einer Kochsalzlösung als umgebendes Medium sicher das abzubildende Objekt beleuchtet werden kann.

Insbesondere kann der nicht-transparente Bereich z. B. geschwärzt sein. Natürlich kann auch der gesamte Bereich am distalen Ende neben dem Deckglas und neben dem transparenten Bereich als nicht-transparenter Bereich (beispielsweise geschwärzt) ausgebildet sein.

Bei der erfindungsgemäßen Schutzhülse können die Mittelpunkte der Krümmungsradien der Innen- und Außenseite zusammenfallen.

Insbesondere ist der rohrförmige Hauptkörper der Schutzhülse aus (z.B. transparentem) Kunststoff gebildet. In gleicher Weise kann das Deckglas aus Kunststoff gebildet sein. Dadurch kann eine sehr kostengünstige Schutzhülse bereitgestellt werden. Die Schutzhülse kann beispielsweise als Tiefziehteil ausgebildet sein, so daß die Schutzhülse als Einwegteil bereitgestellt werden kann.

Insbesondere ist das distale Ende des Hauptkörpers bei der erfindungsgemäßen Schutzhülse verschlossen. Damit kann z.B. das in die Schutzhülse eingesetzte Endoskoprohr vor einem wäßrigen Medium, in das die Schutzhülse samt eingesetztem Endoskoprohr eingetaucht wird, geschützt werden.

Bei der erfindungsgemäßen Schutzhülse kann der Hauptkörper am proximalen Ende ein erstes Verriegelungselement aufweisen, das in Verbindung mit einem zweiten Verriegelungselement am Endoskop eine lösbare Arretierung der Schutzhülse bei einstehendem Endoskoprohr ermöglicht. Insbesondere kann das zweite Verriegelungselement am Endoskoprohr selbst vorgesehen sein.

Die beiden Verriegelungselemente können z.B. so ausgebildet sein, daß ein Bajonett-Verschluß verwirklicht ist. Es ist aber auch jede andere Art einer lösbaren Verbindung bzw. Verriegelung möglich.

Bei der erfindungsgemäßen Schutzhülse kann die Innenseite und/oder die Außenseite des Deckglases entspiegelt sein.

Insbesondere kann der transparente Bereich in zwei orthogonalen Richtungen eine sphärisch gekrümmte, konkave Innenseite, eine sphärisch gekrümmte, konvexe Außenseite sowie eine konstante Dicke aufweisen.

Der Hauptkörper der erfindungsgemäßen Schutzhülse ist bevorzugt starr ausgebildet.

Es wird ferner ein Endoskop mit einem Endoskoprohr, einer im Endoskoprohr angeordneten Abbildungsoptik und einer erfindungsgemäßen Schutzhülse (einschließlich ihrer beschriebenen Weiterbildungen) bereitgestellt, wobei ein distaler Abschnitt des Endoskoprohrs in die Schutzhülse einsteht, die Schutzhülse lösbar am Endoskop befestigt ist und die Abbildungsoptik ein vor dem Deckglas befindliches Objekt als Bild abbildet.

Eine solche Kombination aus Endoskop und Schutzhülse gewährleistet, daß das Endoskop im wäßrigen Medium gute Abbildungseigenschaften aufweist und daß das Endoskoprohr vor dem wäßrigen Medium sowie vor mechanischen Beschädigungen geschützt ist.

Bei dem erfindungsgemäßen Endoskop können die Mittelpunkte der Krümmungsradien der Innen- und Außenseite des Deckglases zusammenfallen und in der Eintrittspupille der Abbildungsoptik liegen. Damit werden sehr gute Abbildungseigenschaften erreicht. Insbesondere ist der Einfluß auf die Abbildung auf das Deckglas minimiert.

Bei dem erfindungsgemäßen Endoskop kann die Abbildungsoptik ein schwenkbar gelagertes Umlenkelement aufweisen, mit dem die Blickrichtung durch das Deckglas einstellbar ist. Das Umlenkelement kann eine die Strahlumlenkung bewirkende Fläche aufweisen. Die Mittelpunkte der Krümmungsradien der Innen- und Außenseite des Deckglases können bevorzugt zusammenfallen und auf der Schwenkachse des Umlenkelementes liegen oder in der die Strahlumlenkung bewirkenden Fläche liegen.

Des weiteren kann die Abbildungsoptik mehrere Linsen aufweisen, wobei die am nähesten am Deckglas positionierte Linse der Abbildungsoptik als Plan-Konvex-Linse ausgebildet ist, deren plane Seite dem Deckglas zugewandt ist.

Das Endoskoprohr ist bevorzugt starr ausgebildet.

Das Endoskop kann eine Beleuchtungseinheit aufweisen, die Beleuchtungslicht auf das vor dem Deckglas befindliche Objekt richten kann. Insbesondere kann das Beleuchtungslicht durch den transparenten Bereich für Beleuchtungslicht bei der Schutzhülse laufen. Dabei ist es bevorzugt, wenn das Beleuchtungslicht senkrecht oder im wesentlichen senkrecht durch den transparenten Bereich hindurchtritt, so daß in vorteilhafter Weise keine oder eine im wesentlichen vernachlässigbare Brechung unabhängig vom an der Außenseite des transparenten Bereichs vorhandenen Medium auftritt. Damit kann z.B. sichergestellt werden, daß selbst bei einer Kochsalzlösung als umgebenes Medium sicher das abzubildende Objekt beleuchtet werden kann.

Es versteht sich, daß die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung beispielsweise anhand der beigefügten Zeichnungen, die auch erfindungswesentliche Merkmale offenbaren, noch näher erläutert. Es zeigen:
- Fig. 1: eine schematische Darstellung der erfindungsgemäßen Schutzhülse im auf ein Endoskoprohr eines Endoskopes aufgeschobenen Zustand;
- Fig. 2: eine schematische Darstellung der Schutzhülse von Fig. 1;
- Fig. 3: eine schematische Darstellung des Endoskops von Fig. 1;
- Fig. 4: eine schematische Darstellung des Deckglases der erfindungsgemäßen Schutzhülse sowie der Abbildungsoptik im distalen Endbereich des Endoskoprohrs;
- Fig. 5: eine weitere Ausführungsform der erfindungsgemäßen Schutzhülse im auf ein Endoskoprohr eines Endoskopes aufgeschobenen Zustand;
- Fig. 6: die erfindungsgemäße Schutzhülse gemäß Fig. 5;
- Fig. 7: das Endoskop gemäß Fig. 5;
- Fig. 8: eine perspektivische Darstellung des distalen Endabschnitts der erfindungsgemäßen Schutzhülse gemäß Fig. 5 und 6, und
- Fig. 9: eine perspektivische Darstellung des distalen Endabschnitts einer weiteren Ausführungsform der erfindungsgemäßen Schutzhülse.

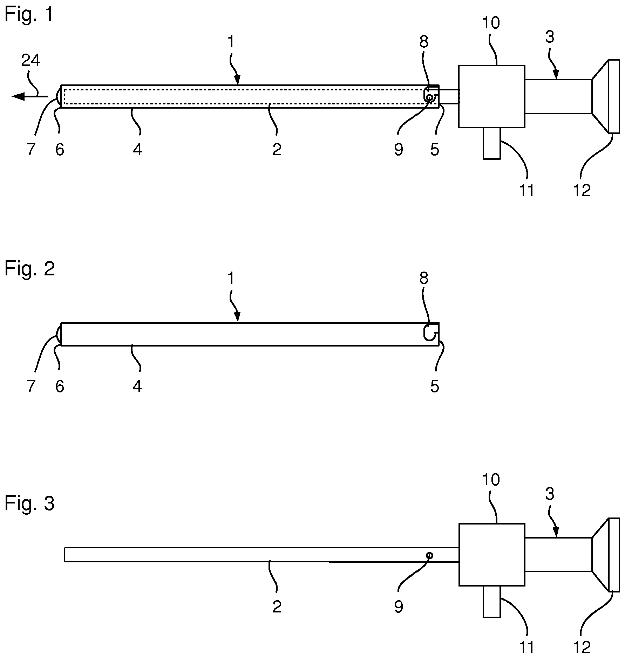

Bei der in Fig. 1 gezeigten Ausführungsform ist die erfindungsgemäße Schutzhülse 1 auf das Endoskoprohr 2 eines Endoskops 3, das insbesondere als Arthroskop ausgebildet sein kann, aufgeschoben.

In Fig. 2 ist die Schutzhülse 1 alleine und in Fig. 3 ist das Endoskop 3 ohne Schutzhülse 1 dargestellt.

Die Schutzhülse 1 weist einen rohrförmigen Hauptkörper 4 auf, der ein offenes proximales Ende 5 und ein distales Ende 6 umfaßt, wobei das distale Ende 6 mittels einem Deckglas 7 verschlossen ist.

Am proximalen Ende 5 des Hauptkörpers 4 ist eine Ausnehmung 8 gebildet, die so geformt ist, daß sie zusammen mit einem am Endoskoprohr 2 befestigten Stift 9 ein lösbares Verbinden von Schutzhülse 1 und Endoskoprohr 2 in Art einer Bajonett-Verbindung ermöglicht.

Das Endoskop 3 weist neben dem Endoskoprohr 2 einen Hauptteil 10 auf, der mit dem proximalen Ende des Endoskoprohrs 2 verbunden ist. An dem Hauptteil 10 ist ein Anschluß 11 ausgebildet, über den Beleuchtungslicht in das Endoskop 3 einkoppelbar ist. Beispielsweise können Lichtleitfasern vom Anschluß 11 bis zum distalen Ende des Endoskoprohrs 2 verlaufen, über die das Licht zur Beleuchtung eines abzubildenden Objektes übertragen wird. Natürlich kann der Anschluß auch weggelassen werden, wenn das gewünschte Beleuchtungslicht beim Einsatz des Endoskopes in anderer Art und Weise bereitgestellt wird.

Ferner ist mit dem Hauptteil 10 noch ein Okular 12 verbunden. Statt des Okulars 12 oder zusätzlich zum Okular 12 kann am Hauptteil 10 ein Anschluß für eine Kamera ausgebildet sein.

Im Endoskoprohr 2 ist eine Abbildungsoptik angeordnet, die ein vor dem Deckglas 7 befindliches Objekt (wenn das Endoskoprohr 2 in der in Fig. 1 gezeigten Art in die Schutzhülse 1 einsteht) als Bild abbildet. Das derart abgebildete Objekt kann dann z.B. in bekannter Art und Weise durch im Endoskoprohr 2 angeordnete Stablinsen bis zum Okular 12 übertragen werden.

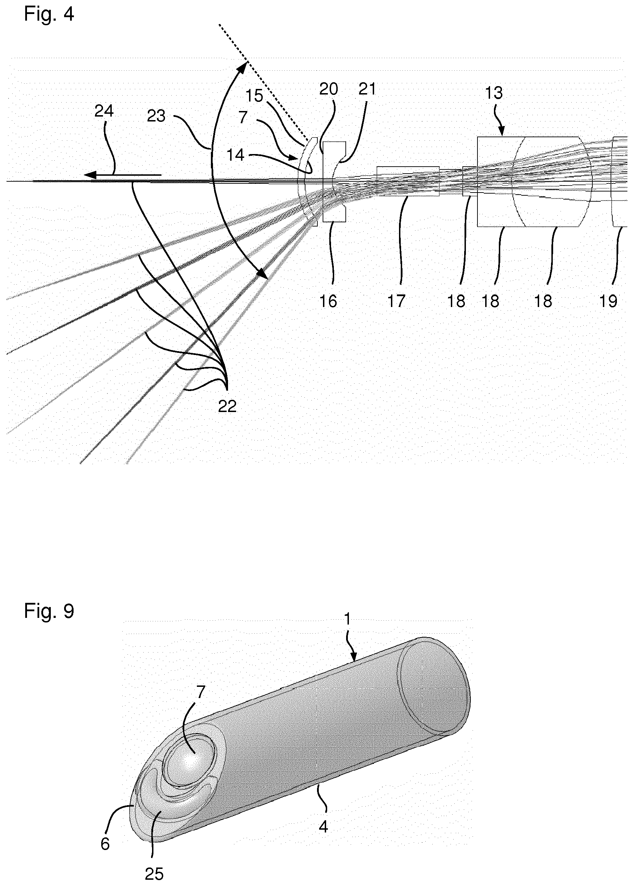

In Fig. 4 sind sowohl die Elemente der Abbildungsoptik 13 im Bereich des distalen Endes des Endoskoprohrs 2 als auch das Deckglas 7 der Schutzhülse 1 schematisch dargestellt.

Das Deckglas 7 weist eine sphärisch gekrümmte, konkave Innenseite 14 und eine sphärisch gekrümmte, konvexe Außenseite 15 auf, wobei die Krümmungsmittelpunkte von Innen- und Außenseite 14, 15 zusammenfallen. Somit ist die Dicke des Deckglases 7 konstant. Bei der hier beschriebenen Ausführungsform liegt die Dicke des Deckglases im Bereich von 0,1-0,3 mm. Sie kann z.B. 0,2 mm betragen. Das Deckglas 7 kann daher auch als Kuppelglas bezeichnet werden.

Die Abbildungsoptik 13 weist eine distale Linse 16, ein optional vorsehbares Prisma 17, Linsen 18 sowie eine Feldlinse 19 auf.

Die distale Linse 16 ist als Plan-Konkav-Linse ausgebildet, wobei ihre plane Seite 20 dem Deckglas 7 zugewandt ist und ihre konkave Seite 21 vom Deckglas 7 abgewandt ist. Die distale Linse 16 ist als Negativlinse ausgebildet.

Zur Verdeutlichung der abbildenden Eigenschaften der Abbildungsoptik 13 sind Lichtbündel 22 eingezeichnet. Um die Darstellung zu vereinfachen, sind jedoch die Lichtbündel 22 nur für den halben Bildwinkel 23 dargestellt.

Durch die beschriebene Ausbildung des Deckglases 7 wird der Vorteil erreicht, daß die von jedem Objektpunkt ausgehenden und für die Bilderzeugung genutzten Strahlen (insbesondere die Hauptstrahlen jedes Lichtbündels) senkrecht bzw. im wesentlichen senkrecht durch das Deckglas 7 hindurchtreten. Daher kann das Deckglas 7 als für die Abbildung im wesentlichen neutrales Element (das im wesentlichen keine optischen Abbildungseigenschaften aufweist) angesehen werden, so daß das Deckglas 7 auch als Nullinse bezeichnet werden kann. Da die für die Bilderzeugung genutzten Strahlen senkrecht bzw. im wesentlichen senkrecht durch das Deckglas 7 hindurchlaufen, tritt in vorteilhafter Weise keine oder eine im wesentlichen vernachlässigbare Brechung unabhängig vom an der Außenseite 15 des Deckglases 7 vorhandenen Medium auf.

So wird z.B. in der Arthroskopie von Knien und Schultergelenken in den meisten Fällen der Operationsbereich mit einer Kochsalzlösung gefüllt. Dabei wird zur Visualisierung des zu operierenden Bereiches ein Endoskop eingesetzt. Durch die erfindungsgemäße Schutzhülse 1 mit dem Deckglas 7 kann der große Bildwinkel von z.B. 105°, wie in Fig. 4 gezeigt, aufrechterhalten bzw. zumindest nahezu aufrechterhalten werden und es tritt nicht mehr eine Verringerung des Bildwinkels auf z.B. 75° auf, wie dies der Fall ist, wenn das Endoskop z.B. ein planes Deckglas aufweist.

Bei der in Verbindung mit Fig. 1 bis 4 beschriebenen Ausführungsform ist die Blickrichtung 24 des Endoskopes 2 parallel zur Längsrichtung des Endoskoprohrs 2. Man spricht hier auch von einer Blickrichtung von 0°.

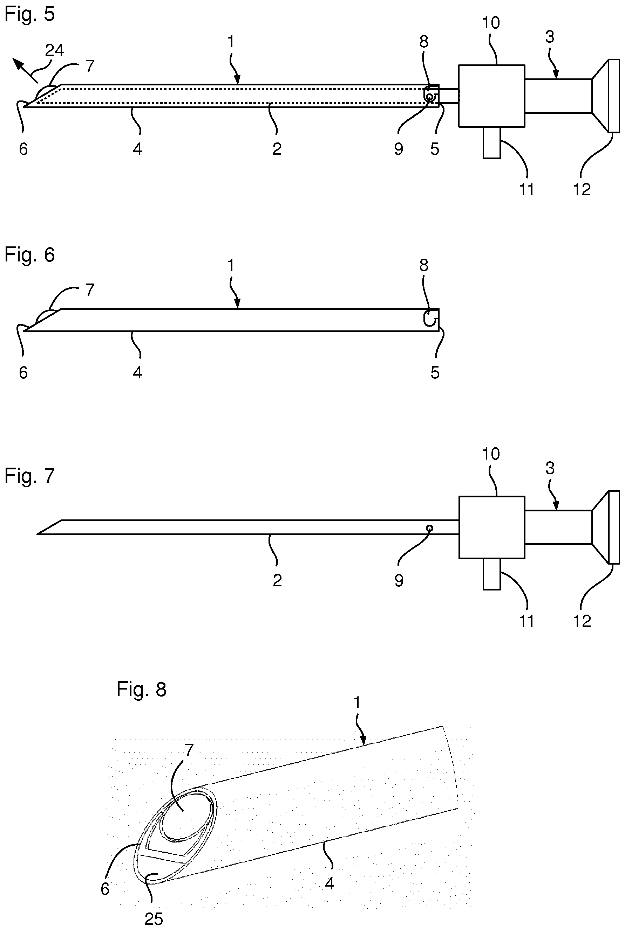

In Fig. 5 bis 7 sind Abwandlungen der erfindungsgemäßen Schutzhülse 1 gezeigt, die für ein Endoskop 3 mit einer Blickrichtung 24 von ca. 45° gezeigt. In diesem Fall ist die Schutzhülse 1 am distalen Ende 6 entsprechend angepaßt. In jedem Fall ist wiederum ein Deckglas 7 mit konstanter Dicke und sphärisch gekrümmter, konkaver Innenseite 14 sowie sphärisch gekrümmter, konvexer Außenseite 15 vorgesehen. Bei einer solchen Blickrichtung ist zwischen Deckglas 7 einerseits und der weiteren Optik im Endoskoprohr 2 andererseits eine Strahlumlenkung zu einer Strahlengangfaltung notwendig. Dafür kann das in Verbindung mit Fig. 4 erwähnte Prisma 17 vorgesehen werden. In diesem Fall ist die distale Linse 16 zwischen Deckglas 7 und Prisma 17 positioniert (bei aufgeschobener Schutzhülse 1). Natürlich kann die distale Linse 16 auch hinter dem Prisma 17 positioniert sein.

Die erfindungsgemäße Schutzhülse 1 weist neben den beschriebenen optischen Vorteilen noch den Zweck des Schutzes des distalen Endes des Endoskoprohrs 2 auf. Insbesondere soll das distale Ende des Endoskoprohrs 2 vor mechanischen Beschädigungen geschützt werden, die bei dem erfindungsgemäßen Einsatz des Endoskopes 3 (z.B. in der Arthroskopie von Knie- und Schultergelenken) leicht auftreten können. Wenn z.B. ein Kontakt mit einem Operationsinstrument auftritt, wird nur die Schutzhülse 1 beschädigt und bleibt das wertvollere Endoskop 3 unversehrt.

Daher ist es insbesondere von Vorteil, wenn die Schutzhülse 1 als kostengünstiges Einwegteil ausgeführt ist. Insbesondere kann die Schutzhülse 1 komplett aus Kunststoff hergestellt sein.

In Fig. 8 ist der distale Endbereich der Schutzhülse 1 gemäß Fig. 5 und 6 perspektivisch dargestellt. Neben dem Deckglas 7 kann die Schutzhülse 1 an ihrem distalen Ende 6 noch einen zweiten transparenten Bereich 25 aufweisen, durch den das Licht zur Beleuchtung aus dem Endoskoprohr 2 in Richtung auf das abzubildende Objekt tritt.

Wie in der perspektivischen Darstellung des distalen Endbereichs einer weiteren Ausführungsform der erfindungsgemäßen Schutzhülse in Fig. 9 gezeigt ist, kann dieser transparente Bereich 25 auch so ausgebildet sein, daß er zumindest in einer Richtung eine sphärisch gekrümmte, konkave Innenseite sowie eine sphärisch gekrümmte, konvexe Außenseite bei konstanter Dicke aufweist. Hier ist der transparente Bereich 25 an die Form des Lichtaustrittes am distalen Ende des Endoskoprohrs 2 angepaßt und weist eine halbkreisförmige Form in Draufsicht auf. Der transparente Bereich 25 kann daher auch als Nullring bzw. Nullteilring bezeichnet werden.

Das Deckglas 7 und gegebenenfalls der transparente Bereich 25 können z.B. als Tiefziehteil ausgelegt sein. Insbesondere kann die gesamte Schutzhülse 1 als Tiefziehteil ausgelegt sein. Ferner kann das distale Ende 6 des rohrförmigen Hauptkörpers 4 neben dem Deckglas 7 und gegebenenfalls neben dem transparenten Bereich 25 geschwärzt sein, um unerwünschte Reflexe zu vermindern. Des weiteren kann die Innen- und/oder Außenseite 14, 15 des Deckglases 7 mit einer Antireflexionsbeschichtung versehen sein.

Das Deckglas 7 ist bevorzugt so ausgelegt, daß die Mittelpunkte der beiden Krümmungsradien von Innen- und Außenseite 14, 15 mit der Lage der Eintrittspupille der Abbildungsoptik 13 zusammenfallen, wenn die Schutzhülse 1 in vorbestimmter Weise mit dem Endoskop 3 verbunden ist, wie in Fig. 1 und 5 gezeigt ist. Die Lage der Eintrittspupille wird durch die Schnittpunkte der Hauptstrahlen (mittlerer Lichtstrahl der Lichtbündel 22) bestimmt und ist idealer Weise ein Punkt. Durch das Vorhandensein von Pupillenaberationen im realen System ist die Eintrittspupille meist leicht gekrümmt und daher nicht genau an einem Punkt zu finden. In diesem Fall können die Mittelpunkte der Krümmungen von Innen- und Außenseite 14 und 15 z.B. in der Mitte der verschiedenen Schnittpunkte liegen.

Bei den in Fig. 8 und 9 gezeigten Ausführungsformen kann der rohrförmige Hauptkörper 4 jeweils transparent ausgebildet sein.

Natürlich kann die erfindungsgemäße Schutzhülse 1 auch für Endoskope mit variabler Blickrichtung bereitgestellt werden. Bei diesen Endoskopen weist die Abbildungsoptik 13 ein schwenkbare Umlenkelement auf, mit dem die Blickrichtung einstellbar ist.

## Patentansprüche

1. Schutzhülse für ein ein Endoskoprohr aufweisendes Endoskop,
wobei die Schutzhülse (1) einen rohrförmigen Hauptkörper (4) zur Aufnahme eines distalen Abschnitts des Endoskoprohres (2) umfasst,
wobei der Hauptkörper (4) ein offenes proximales Ende (5) und ein Deckglas (7) am distalen Ende aufweist,
das Deckglas (7) eine sphärisch gekrümmte, konkave Innenseite (14), eine sphärisch gekrümmte, konvexe Außenseite (15) sowie eine konstante Dicke aufweist, und wobei am distalen Ende (6) des Hauptkörpers (4) ein vom Deckglas (7) beabstandeter zweiter transparenter Bereich (25) für Beleuchtungslicht des Endoskopes (3) vorgesehen ist und der Bereich zwischen dem Deckglas und dem zweiten transparenten Bereich am distalen Ende als nicht-transparenter Bereich ausgebildet ist, **dadurch gekennzeichnet, dass** der zweite transparente Bereich (25) in zumindest einer Richtung eine sphärisch gekrümmte, konkave Innenseite, eine sphärisch gekrümmte, konvexe Außenseite sowie eine konstante Dicke aufweist.

2. Schutzhülse nach Anspruch 1, **dadurch gekennzeichnet, dass** die Mittelpunkte der Krümmungsradien der Innen- und Außenseite (14, 15) des Deckglases zusammenfallen.

3. Schutzhülse nach Anspruch 1 oder 2, **dadurch gekennzeichnet, dass** der rohrförmige Hauptkörper (4) aus Kunststoff gebildet ist.

4. Schutzhülse nach einem der obigen Ansprüche, **dadurch gekennzeichnet, dass** das Deckglas (7) aus Kunststoff gebildet ist.

5. Schutzhülse nach einem der obigen Ansprüche, **dadurch gekennzeichnet, dass** das distale Ende (6) des Hauptkörpers (4) verschlossen ist.

6. Schutzhülse nach einem der obigen Ansprüche, **dadurch gekennzeichnet, dass** der Hauptkörper (4) am proximalen Ende ein erstes Verriegelungselement (8) aufweist, das in Verbindung mit einem zweiten Verriegelungselement am Endoskop (3) eine lösbare Arretierung der Schutzhülse (1) bei einstehendem Endoskoprohr (2) ermöglicht.

7. Schutzhülse nach einem der obigen Ansprüche, **dadurch gekennzeichnet, dass** die Innenseite (14) und/oder die Außenseite (15) des Deckglases (7) entspiegelt sind bzw. ist.

8. Endoskop mit
einem Endoskoprohr (2),
einer im Endoskoprohr (2) angeordneten Abbildungsoptik (13) und
einer Schutzhülse (1) nach einem der obigen Ansprüche,
wobei ein distaler Abschnitt des Endoskoprohrs (2) in die Schutzhülse (1) einsteht,
die Schutzhülse (1) lösbar am Endoskop (3) befestigt ist und
die Abbildungsoptik (13) ein vor dem Deckglas (7) befindliches Objekt als Bild abbildet.

9. Endoskop nach Anspruch 8, bei dem die Mittelpunkte der Krümmungsradien der Innen- und Außenseite (14, 15) des Deckglases (7) zusammenfallen und in der Entrittspupille der Abbildungsoptik (13) liegen.

10. Endoskop nach Anspruch 8, bei dem die Abbildungsoptik (13) ein schwenkbar gelagertes Umlenkelement aufweist, mit dem die Blickrichtung durch das Deckglas (7) einstellbar ist.

11. Endoskop nach Anspruch 10, bei dem die Mittelpunkte der Krümmungsradien der Innen- und Außenseite (14, 15) des Deckglases (7) zusammenfallen und auf der Schwenkachse des Umlenkelementes liegen.

12. Endoskop nach Anspruch 10 oder 11, bei dem das Umlenkelement eine eine Strahlumlenkung bewirkende Fläche aufweist, die Mittelpunkte der Krümmungsradien der Innen- und Außenseite (14, 15) zusammenfallen und in der die Strahlumlenkung bewirkenden Fläche liegen.

13. Endoskop nach einem der Ansprüche 8 bis 12, bei dem die Abbildungsoptik (13) mehrere Linsen (16, 18, 19) aufweist, wobei die am nähesten am Deckglas (7) positionierte Linse (16) der Abbildungsoptik (13) als Plan-Konvex-Linse (16) ausgebildet ist, deren plane Seite (20) dem Deckglas (7) zugewandt ist.

## Claims

1. Protective sleeve for an endoscope comprising an endoscope tube,
wherein the protective sleeve (1) includes a tubular main body (4) for receiving a distal portion of the endoscope tube (2),
wherein the main body (4) comprises an open proximal end (5) and a cover glass (7) at the distal end, the cover glass (7) comprises a spherically curved, concave inside (14), a spherically curved, convex outside (15) and a constant thickness
and wherein
a second transparent region (25) spaced apart from the cover glass (7) for illumination light of the endoscope (3) is provided at the distal end (6) of the main body (4) and the region between the cover glass and the second transparent region is formed as a non-transparent region at the distal end, **characterized in that** the second transparent region (25) comprises in at least one direction a spherically curved, concave inside, a spherically curved, convex outside as well as a constant thickness.

2. Protective sleeve according to claim 1, **characterized in that** the centers of the radii of curvature of the inside and outside (14, 15) of the cover glass coincide.

3. Protective sleeve according to claim 1 or 2, **characterized in that** the tubular main body (4) is made of plastic.

4. Protective sleeve according to any of the above claims, **characterized in that** the cover glass (7) is made of plastic.

5. Protective sleeve according to any one of the above claims, **characterized in that** the distal end (6) of the main body (4) is closed.

6. Protective sleeve according to any of the above claims, **characterized in that** the main body (4) comprises a first locking element (8) at the proximal end which, in conjunction with a second locking element at the endoscope (3) allows to detachably lock the protective sleeve (1) when the endoscope tube (2) is retracted.

7. Protective sleeve according to any of the above claims, in which the inside (14) and/or the outside (15) of the cover glass (7) is or are antireflection-coated.

8. Endoscope having
an endoscope tube (2),
imaging optics (13) arranged in the endoscope tube (2) and
a protective sleeve (1) according to any of the above claims,
wherein a distal portion of the endoscope tube (2) projects into the protective sleeve (1),
the protective sleeve (1) is detachably attached to the endoscope (3) and
the imaging optics (13) images an object located in front of the cover glass (7) as an image.

9. Endoscope according to claim 8, in which the centers of the radii of curvature of the inside and outside (14, 15) of the cover glass (7) coincide and lie in the entrance pupil of the imaging optics (13).

10. Endoscope according to claim 8, wherein the imaging optics (13) comprises a pivotally mounted deflection element, with which the viewing direction is adjustable through the cover glass (7).

11. Endoscope according to claim 10, in which the centers of the radii of curvature of the inside and outside (14, 15) of the cover glass (7) coincide and lie on the pivot axis of the deflection element.

12. Endoscope according to claim 10 or 11, wherein the deflection element comprises a surface effecting a beam deflection, the centers of the radii of curvature of the inside and outside (14, 15) coincide and lie in the surface effecting the beam deflection.

13. Endoscope according to any of claims 8 to 12, wherein the imaging optics (13) comprises a plurality of lenses (16, 18, 19), wherein the lens (16) positioned closest to the cover glass (7) of the imaging optics (13) is formed as a plano-convex lens (16), which flat side (20) faces the cover glass (7).

## Revendications

1. Douille de protection pour un endoscope présentant un tube d'endoscope,
dans laquelle la douille de protection (1) comprend un corps principal tubulaire (4) destiné à recevoir une partie distale d'un tube d'endoscope (2),
dans laquelle le corps principal (4) présente une extrémité proximale ouverte (5) et un verre de recouvrement (7) à l'extrémité distale,
le verre de recouvrement (7) présente un côté intérieur concave à courbure sphérique (14), un côté extérieur convexe à courbure sphérique (15) ainsi qu'une épaisseur constante, et
dans laquelle il est prévu à l'extrémité distale (6) du corps principal (4) une deuxième région (25) espacée du verre de recouvrement (7) transparente pour la lumière d'éclairage de l'endoscope (3) et la région comprise entre le verre de recouvrement et la deuxième région transparente à l'extrémité distale est une région non transparente,
**caractérisée en ce que** la deuxième région transparente (25) présente dans au moins une direction un côté intérieur concave à courbure sphérique, un côté extérieur convexe à courbure sphérique ainsi qu'une épaisseur constante.

2. Douille de protection selon la revendication 1, **caractérisée en ce que** les points centraux des rayons de courbure des côtés intérieur et extérieur (14, 15) du verre de recouvrement coïncident.

3. Douille de protection selon la revendication 1 ou 2, **caractérisée en ce que** le corps principal tubulaire (4) est formé en matière plastique.

4. Douille de protection selon l'une quelconque des revendications précédentes, **caractérisée en ce que** le verre de recouvrement (7) est formé en matière plastique.

5. Douille de protection selon l'une quelconque des revendications précédentes, **caractérisée en ce que** l'extrémité distale (6) du corps principal (4) est fermée.

6. Douille de protection selon l'une quelconque des revendications précédentes, **caractérisée en ce que** le corps principal (4) présente à l'extrémité proximale un premier élément de verrouillage (8) qui permet, en liaison avec un deuxième élément de verrouillage sur l'endoscope (3), un blocage libérable de la douille de protection (1) lorsque le tube d'endoscope (2) est engagé.

7. Douille de protection selon l'une quelconque des revendications précédentes, **caractérisée en ce que** le côté intérieur (14) et/ou le côté extérieur (15) du verre de recouvrement (7) est/sont antireflet.

8. Endoscope avec
un tube d'endoscope (2),
une optique d'imagerie (13) disposée dans le tube d'endoscope (2),
une douille de protection (1) selon l'une quelconque des revendications précédentes,
dans lequel une partie distale du tube d'endoscope (2) s'engage dans la douille de protection (1),
la douille de protection (1) est fixée de façon libérable à l'endoscope (3) et
l'optique d'imagerie (13) forme une image d'un objet se trouvant devant le verre de recouvrement (7).

9. Endoscope selon la revendication 8, dans lequel les points centraux des rayons de courbure des côtés intérieur et extérieur (14, 15) du verre de recouvrement (7) coïncident et sont situés dans la pupille d'entrée de l'optique d'imagerie (13).

10. Endoscope selon la revendication 8, dans lequel l'optique d'imagerie (13) présente un élément de déviation monté de façon pivotante, avec lequel la direction du regard à travers le verre de recouvrement (7) peut être réglée.

11. Endoscope selon la revendication 10, dans lequel les points centraux des rayons de courbure des côtés intérieur et extérieur (14, 15) du verre de recouvrement (7) coïncident et sont situés sur l'axe de pivotement de l'élément de déviation.

12. Endoscope selon la revendication 10 ou 11, dans lequel l'élément de déviation présente une face provoquant une déviation des rayons, les points centraux des rayons de courbure des côtés intérieur et extérieur (14, 15) coïncident et sont situés dans la face provoquant la déviation des rayons.

13. Endoscope selon l'une quelconque des revendications 8 à 12, dans lequel l'optique d'imagerie (13) présente plusieurs lentilles (16, 18, 19), dans lequel la lentille (16) de l'optique d'imagerie (13) positionnée le plus près du verre de recouvrement (7) est une lentille plan-convexe (16), dont le côté plan (20) est tourné vers le verre de recouvrement (7).
